## Europäisches Patentamt
## European Patent Office
## Office européen des brevets

(19)

(11) Publication number: **0 210 734**
**A1**

(12)

# EUROPEAN PATENT APPLICATION

(21) Application number: 86304472.3

(22) Date of filing: 11.06.86

(51) Int. Cl.⁴: **A 01 N 63/00**
**C 12 N 1/20**
**//(C12N1/20, C12R1:39)**

(30) Priority: 14.06.85 AU 1031/85

(43) Date of publication of application:
04.02.87 Bulletin 87/6

(84) Designated Contracting States:
DE FR GB NL

(71) Applicant: BURNS, PHILIP & COMPANY LIMITED
Pitt Street
Sydney New South Wales(AU)

(71) Applicant: THE MINISTER FOR THE DEPARTMENT OF
AGRICULTURE OF THE STATE OF NEW SOUTH WALES,
COMMONWEALTH OF AUSTRALIA, of
Biological and Chemical Research Institute Corner
Pemberton Street & Victoria Road
Rydalmere New South Wales(AU)

(72) Inventor: Fahy, Peter Biological and Chemical Res. Inst.
Department of Agriculture Victoria Road
Rydalmere New South Wales 2116(AU)

(74) Representative: Crampton, Keith John Allen et al,
D YOUNG & CO 10 Staple Inn
London WC1V 7RD(GB)

(54) Mushroom blotch control agent.

(57) A control agent for use in the control of blotch on mush-
rooms comprises an isolated strain of *P. fluorescens* designated NCIB 12087, NCIB 12088, NCIB 12089 or NCIB 12090.

The strains are selected by

(i) selecting the prime-dominant strain from visually
healthy sporophores obtained from crops showing less than
one percent visible blotch despite growth at a relative humidity exceeding 80%;

(ii) combining each strain obtained in step (i) with a
*Pseudomonas tolaasii* pathogen in various ratios of *P. fluorescens* to *P. tolaasii* and retaining those *P. fluorescens* strains
which give a consistent reduction in tissue browning at *P.
fluorescens/P. tolaasii* ratios of 5/1 or less;

(iii) eliminating from the *P. fluorescens* strains retained in
step (ii) those that inhibit or distort mycelial growth; and

(iv) selecting rifampicin-resistant mutants from the
strains remaining after step (iii).

1

# MUSHROOM BLOTCH CONTROL AGENT

This invention relates to the control of mushroom blotch and to a biological control agent for use in the method.

Mushroom growing is a highly sophisticated and capital intensive industry. Bacterial blotch is a serious disease of mushrooms causing a 3-5% spoilage loss among cultivated mushrooms such as Agaricus bisporus and A. bitorquis.

The pathogen causing blotch, Pseudomonas tolaasii, lives on the surface of mushroom caps growing on nutrients which leak from the mushroom tissue. If prolonged surface wetness occurs and other bacteria present are not antagonistic to growth of the pathogen, then the pathogen multiplies and spreads across the cap becoming the dominant organism and growing to levels of up to 50 million cells per cap without any visible disease symptoms occurring.

If caps remain wet, either intermittently or for a prolonged period, then a toxic chemical produced by the pathogen is produced in sufficient concentrations to damage mushroom cells causing a greatly increased leakage of nutrients and initiating a defence mechanism of the mushroom which results in visible browning of the cap as the mushroom tissue attempts to seal off further invasion by the pathogen. Under conditions favourable to the pathogen, bacteria can double in population in less than 1 hour and in less than two hours visible disease can occur apparently spontaneously in a healthy mushroom crop or during transport or storage.

It has previously been proposed to control blotch by introducing direct innoculation of the peat or compost of a mushroom crop with an antagonist. A suitable antagonist is a bacterium naturally occurring on mushrooms and harmless to humans but which is competitive with the pathogen and able to suppress pathogen population levels.

The present invention resides in the isolation of new strains of the organism P. fluorescens and the discovery that selected of the isolated strains of organism are particularly effective as biological agents for mushroom blotch.

According to one aspect the invention provides a control agent for use in the control of blotch on mushrooms comprising at least one isolated strain of P. fluorescens designated NCIB 12087, NCIB 12088, NCIB 12089 and/or NCIB 12090. The strains identified as above were deposited in the "National Collection of Industrial Bacteria" culture collection at Aberdeen, Scotland on 20th May, 1985 under File No. PAT/45 and are more particularly described hereinafter.

According to a second aspect the invention provides a method for the control of blotch on a mushroom comprising treating the mushroom or its environment with a biological control agent comprising predominantly at least one strain of P. fluorescens designated NCIB 12087, 12088, 12089 or 12090 or a mixture of said strains.

Of these strains the most effective is believed to be NCIB 12089.

The biological control agents according to the invention were selected by screening several hundred strains of bacteria isolated from mushroom tissue against a range of concentrations of pathogen directly on mushroom caps.

The method involved, which consititute another aspect of the present invention, comprises

(i)     selecting the prime dominant strain from visually healthy sporophores obtained from crops showing less than one percent visible blotch despite growth at a relative humidity exceeding 80%;

(ii)    combining each strain obtained in step (i) with a Pseudomonas tolaasii pathogen in various ratios of P. fluorescens to P. tolaasii and retaining those P. fluorescens strains which give a consistent reduction in tissue browning at P. fluorescens/P. tolaasii ratios of 5/1 or less;

(iii)   eliminating from the P. fluorescens strains retained in step (ii) those that inhibit or distort mycelial growth;  and

(iv)    selecting rifampicin-resistant mutants from the strains remaining after step (iii).

This special method was developed because it was found that, while a number of bacteria that grow normally on mushroom caps may appear to have a detrimental effect on the pathogen, many of those stop mycelial growth, others grow poorly on mushroom tissue and cannot compete with the pathogen, and others are merely the pathogen itself in a saprophytic state.

In a practical method of carrying out the selection in accordance with the invention, the strains according to the invention were isolated in the following manner:

Stage 1: Only the prime dominant strain from visually healthy button size sporophores (caps) 10-20 mm diameter were selected. Sporophores were plated within 4 hours of harvest. Sporophores were selected from a commercial farm in the second to third flush of growth from a crop showing less than one per cent visible blotch despite relative humidity exceeding 80%. Sporophores were washed in sterile water for 30 minutes and the washate plated onto a non-selective bacterial growth medium such as nutrient agar. Sporophores were from a pure white strain of Agaricus bisporus.

A selective growth medium was then used to reselect potential strains from one of the dominant colony types of Pseudomonas fluorescens present on the caps. The medium (hereinafter referred to as "APA") is a variation of King's B. medium developed by Sands, D.C. and Rovira, A.O. (Appl. Microbiol. 20, 513, 1970) and was used as the base medium for all growth media for selection of antagonist and pathogen.

Stage 2: Each strain selected in stage 1 was then screened against an effective Pseudomonas tolaasii pathogen selected on King's medium B (J. Lab Clin. Med. 44, 301) from a freshly diseased sporophore to minimise the high frequency non pathogenic mutant forms present. Candidate strains were mixed as water suspensions with the pathogen in different ratios and one drop of mixture inoculated onto a healthy, freshly picked sporophore maintained at 18-20°C in a humid chamber and incubated for 24 hours. A range of screening ratios was employed starting with a $10^8$ viable cells/ml suspension of candidate strain and a test strain/pathogen ratios of 1/1; 2/1; 5/1; 10/1. Screening was replicated. Only strains giving a consistent reduction in tissue browning at candidate/pathogen ratios of 5/1 or less were retained. Surprisingly the most effective strains for blotch control were amongst the least effective strains retained from this test, giving only a slight reduction in tissue browning at ratios of 5/1 and 2/1.

Stage 3: Candidate strains from stage 2 were rescreened against a wide selection (for example 8-10) of pathogenic <u>Pseudomonas</u> <u>tolaasii</u> strains including the "ginger" type and candidates not effective against all strains were excluded.

Stage 4: Potential antagonists from stage 3 were next screened against <u>Agaricus</u> <u>bisporus</u> mycelial growth on 1/4 strength potato dextrose agar to eliminate candidates that inhibit or change mycelial growth. That was done in two ways: (a) by mixing candidate suspension directly with mycelium (greater than $10^8$ viable cells/ml) and plating a 5mm diameter core on the centre of a Petri dish. The procedure was replicated and growth rate and morphology compared to uninoculated controls. (b) By streaking candidate directly on plate, separated from growing mycelium by 20 mm, and observing for any signs of growth inhibition or distortion. All candidates that inhibited or distorted growth were rejected.

Stage 5: Remaining candidates from stage 4 were screened against growing mushrooms by inoculating at $10^{10}$ cells/square metre of bed at spawning and casing. Trials were of sufficient replicated design to detect yield inhibition of 10% or more. Strains showing a significant yield inhibition or ineffective control were rejected. An effective strain of the pathogen should be applied at $10^9$ cells/square metre at spawning and casing on a crop grown at greater than 80% relative humidity.

Stage 6: Remaining effective candidate strains from stage 5 were screened against a wide selection (for example 8-10) of pathogenic Pseudomonas tolaasii strains in a similar test design as used in stage 5. Strains not effective against all isolates were rejected.

Stage 7: All effective strains from stage 6 were then freeze dried to minimise loss of antagonist factors by mutation.

Stage 8: Effective candidates from stage 7 were screened for colonisation of mushroom sporophores by selecting rifampicin resistant mutants on King's B + 100 micrograms per millilitre rifampicin. Colonies of unaltered growth rate, morphology and effectiveness were retained. Colonisation was traced by use of a selective medium containing 80 micrograms per millilitre rifampicin. Pathogenic strains of P. tolaasii were traced using a selective pathogen medium consisting of the APA medium to which is added 2.33 micrograms per millilitre of colistin methane sulfonate (activity 13649 units/mg, Sigma Chemical Co. no. C-1511) plus 4.0 micrograms per millilitre of oxytetrocycline hydrochloride (greater than 90% active). Colistin methane sulfonate is also known as colmycin and polymyxin E. Effective antagonists retain a ratio of 10/1 or better (antagonist/pathogen) on sporophore surfaces throughout cropping.,

The selected strains of P. fluorescens according to the invention have the following characteristics.

| Character | Days Incubation at 27°C | Strain No. A | B | C | D |
|---|---|---|---|---|---|
| Fluorescence | 1 | + | + | + | + |
| Levan production | 3 | − | − | − | − |
| Arginine dihydrolase | 3 | + | + | + | + |
| Nitrate reduction | 7 | − | − | − | − |
| Gelatin hydrolysis | 3 | + | + | + | + |
| Carbon source utilisation | | | | | |
| control | 7 | − | − | − | − |
| Ethanol 0.6% | 7 | + | + | + | + |
| m-tartrate 0.3% | 7 | + | + | + | + |
| Adonitol 0.3% | 7 | + | + | + | + |
| Mannitol 0.3% | 7 | + | + | + | + |
| Sorbitol 0.3% | 7 | + | + | + | + |
| Trehalose 0.3% | 7 | + | + | + | + |
| Sucrose 0.3% | 7 | − | − | − | − |

A = NCIB 12088; B = NCIB 12089; C = NCIB 12090 and

D = NCIB 12087

COLONY MORPHOLOGY on King's medium B (27°C)

NCIB 12088

24 hours: cream colonies 0.7-1.0 mm diameter, fluorite green fluorescence at 366 nm with slight yellow diffusable pigment.

72 hours: 4 mm, circular, low convex surface, slightly beaten copper appearance, translucent with entire edge.

96 hours:    5-7mm, as above, butryous,

emulsifiable.

Do not produce a white line on King's medium B

in 48 hours when compared with a pathogenic

strain of King's medium B.

NCIB 12089

Colony morphology as for NCIB 12088 with

exception that a second colony type present in

culture.

Type 2 colony differs from type 1 in ability to

produce a white line of precipitate in the test

of Wong and Preece (1979).

NCIB 12090

Colony morphology same as NCIB 12088.  White

line positive.

NCIB 12087

Colony morphology similar to NCIB 12088 with

exception:  at 72 hr colony centre more opaque

than edge and at 96 hr edge very slightly erose,

butryous to slightly viscid.  White line

positive.

Methods:

As described in:  Fahy, P.C. (1981).  The

taxonomy of the bacterial plant pathogens of

mushroom culture.  Mushroom Science 11, 293-311.

White line test:  Wong, W.C. and Preece, T.F.

(1979).  Identification of rapid pitting test.

J., appl. Bact. 47, 401-407.

Colony morphology: Topley and Wilson's Principles of Bacteriology and Immunology 5th Edition, Chapter 13 by G.S. Wilson and A.A. Miles. Edward Arnold (Publishers) London 1964. In addition all cultures are non-pathogenic to mushrooms and all prevent development of bacterial blotch symptoms when inoculated onto mushroom cap surfaces at a ratio of 5/1 with a pathogenic isolate of Pseudomonas tolaasii at $10^8$ viable cells/ml.

All isolates are members of a single group (phenon) as described by Fahy, 1981.

Table 1 shows a comparison of biological control of blotch using the above strains and other strains of P. fluorescens for comparison.

In trial 1, six antagonists used had no effect on growth of mycelium in plant culture but the isolate identified as 8-2 markedly inhibited mycelial growth and inhibited yield of healthy mushrooms in the in vivo trial. The isolates of the invention were significantly better than other tested strains. The preferred strains yielded approximately 500% more healthy mushrooms than diseased trays.

Additional applications of antagonist did not significantly improve disease control. The antagonists were also applied in all combinations without the pathogen and no inhibition of yield was evident although a noticeable non-significant increase in yield was observed.    Table 2 shows that application of combinations of the preferred strains is more effective than use of the strains singly.

Strain NCIB 12088 is comparable with strain NCIB 12089 in terms of effectiveness.

A method of treating mushrooms by means of the control agent according to the invention will now be further described by way of example only.

A pure liquid of the isolated strain is prepared as a pure liquid culture grown in a medium containing for example glucose as the energy source. For example, a concentrate containing 10,000 million cells per ml is grown in a medium initially containing:

| (a) | glucose | 3.0 | g/l |
|-----|---------|-----|-----|
| (b) | yeast extract | 1.0 | g/l |
| (c) | $KH_2PO_4$ | 4.53 | g/l |
| (d) | $Na_2HPO_4.12H_2O$ | 9.50 | g/l |
| (e) | $NH_4Cl$ | 1.0 | g/l |
| (f) | $MgSO_4.7H_2O$ | 0.5 | g/l |
| (g) | ferric ammonium citrate | 0.05 | g/l |
| (h) | $CaCl_2$ | 10.005 | g/l |

Other of the isolated strains or mixtures thereof may be substituted for NCIB 12089 in the above formulation and/or may be grown in any suitable medium.

For preference the concentrate is applied (at a dilution of between 10 ml/litre and 1 ml/litre) to mushroom compost, casing or tissue to achieve a coverage equivalent to 2 ml of concentrate/sq metre of bed surface.    The concentrate may be applied by spraying onto packed compost or casing with normal water.

A minimum of two applications is desirable for preventative control of blotch.

(1)    The first treatment is best applied to freshly pasteurised compost that has cooled to a surface temperature of from $33^{\circ}$ to $30^{\circ}C$ within about 12 hours of reaching that temperature.  For preference the bed surfaces are sprayed immediately prior to or after spawning with mushroom mycelium.

(2)    The second treatment is best applied to freshly cased compost within four hours of casing preparation and preferably by spraying bed surfaces immediately after casing.

(3)     Additional applications may be made in normal waterings at the onset of pinning and after picking of each flush to a maximum of 4 additional applications.

In a preferred application method, the mushroom blotch control agent is applied during the cooling down phase of compost peak heating as soon as the bed surface temperature reaches $33^{\circ}$ - $30^{\circ}$ and before the compost is removed for spawning, the inoculum being injected as a fine spray into the air conditioning ducting, or as an air-blasted fog into the room or any similar dispersal method.

Other stages at which the inoculum can be applied are to growing spawn, to casing mixture, to revitalised or pasteurised casing immediately after surface drops below $33^{\circ}$ or to mushroom sporophores.

## TABLE 1

| Treatment | Diseased Mushrooms g/tray | ln(g/ tray + 1) | Healthy Mushrooms g/tray | ln(g/ tray+1 |
|---|---|---|---|---|
| **TRIAL 1** | | | | |
| nil pathogen, nil antagonist | 160 | 5.08 | 544 | 6.30 |
| pathogen, nil antagonist | 458 | 6.11 | 64 | 4.18 |
| pathogen plus antagonist | | | | |
| (4-1-2) | 402 | 6.00 | 186 | 5.23 |
| " " " (4-2-2) | 410 | 6.02 | 43 | 3.78 |
| " " " (5-1-2) | 520 | 6.28 | 116 | 4.76 |
| " " " (8-2) | 590 | 6.40 | 24 | 3.20 |
| " " " (NCIB 12087) | 410 | 6.02 | 371 | 5.92 |
| " " " (C12) | 435 | 6.10 | 173 | 5.16 |
| " " " (NCIB 12089) | 231 | 5.45 | 313 | 5.75 |
| SE | | 0.18 | | 0.32 |
| LSD (p=0.05) | | 0.53 | | 0.93 |

Mean Yield of Mushrooms

TABLE 2

| Treatment | Mean Yield of Mushrooms | | | |
| --- | --- | --- | --- | --- |
| | Diseased Mushrooms | | Healthy Mushrooms | |
| | g/tray | ln(g/ tray + 1) | g/tray | ln(g/ tray+1 |
| **TRIAL 2** | | | | |
| nil pathogen, nil antagonist | 24 | 3.21 | 327 | 5.79 |
| pathogen, nil antagonist | 358 | 5.87 | 125 | 4.84 |
| pathogen plus antagonist | | | | |
| (NCIB 12090) | 199 | 5.30 | 232 | 5.45 |
| (NCIB 12089) | 338 | 5.82 | 193 | 5.27 |
| (NCIB 12087) | 214 | 5.37 | 229 | 5.44 |
| (NCIB 12090 + NCIB 12089) | 150 | 5.02 | 273 | 5.61 |
| (NCIB 12090 + NCIB 12087) | 182 | 5.21 | 269 | 5.60 |
| (NCIB 12089 + NCIB 12087) | 171 | 5.15 | 326 | 5.78 |
| (NCIB12090 + NCIB12089 + NCIB12087) | 66 | 3.21 | 362 | 5.89 |
| SE | | 0.67 | | 0.19 |
| LSD (p=0.05) | | 1.89 | | 0.54 |

CLAIMS                                    0210734

1.      A control agent for use in the control of blotch on mushrooms
comprising one or more isolated strains of P. fluorescens designated
NCIB 12087, NCIB 12088, NCIB 12089 and/or NCIB 12090.

2.      A control agent for use in the control of blotch on mushrooms
comprising an isolated strain of P. fluorescens identified as NCIB 12089.

3.      A control agent for use in the control of blotch on mushrooms
comprising an isolated strain of P. fluorescens identified as NCIB 12088.

4.      A method of treatment of a mushroom for the control of blotch
thereon comprising the step of treating the mushroom or its environment
with a control agent as claimed in Claim 1, 2 or 3.

5.      A method for control of blotch on mushrooms comprising inoculating
a peat or compost suitable for growing mushrooms with an antagonist
effective against mushroom blotch, the innoculation being conducted during
the cooling down phase of compost peak heating.

6.      A method for selecting strains of P. fluorescens for use in the control
of mushroom blotch, comprising

(i)     selecting the prime dominant strain from visually healthy sporophores
        obtained from crops showing less than one percent visible blotch
        despite growth at a relative humidity exceeding 80%;

(ii)    combining each strain obtained in step (i) with a Pseudomonas tolaasii
        pathogen in various ratios of P. fluorescens to P. tolaasii and retaining
        those P. fluorescens strains which give a consistent reduction in
        tissue browning at P. fluorescens/P. tolaasii ratios of 5/1 or less;

(iii)   eliminating from the P. fluorescens strains retained in step (ii) those
        that inhibit or distort mycelial growth;  and

(iv)    selecting rifampicin-resistant mutants from the strains remaining
        after step (iii).

7.      Mushroom or mushroom growing materials that have been treated
with a control agent as claimed in any one of Claims 1 to 3.

European Patent
Office

**EUROPEAN SEARCH REPORT**

0210734

Application number

EP 86 30 4472

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | JOURNAL OF APPLIED BACTERIOLOGY, vol. 35, 1972, pages 439-442, New York, US; N.G. NAIR et al.: "Bacteria antagonistic to Pseudomonas tolaasii and their control of brown blotch of the cultivated mushroom Agaricus bisporus" | 1-7 | A 01 N 63/00<br>C 12 N 1/20 //<br>(C 12 N 1/20<br>C 12 R 1:39 ) |

-----

**TECHNICAL FIELDS SEARCHED (Int. Cl.4)**

A 01 N
C 12 N

The present search report has been drawn up for all claims

| Place of search | Date of completion of the search | Examiner |
|---|---|---|
| THE HAGUE | 29-10-1986 | DECORTE D. |